# EUROPÄISCHE PATENTSCHRIFT

(11) **EP 2 304 088 B1**
(45) Veröffentlichungstag und Bekanntmachung des Hinweises auf die Patenterteilung: **28.11.2012**
(21) Anmeldenummer: 09771988.4
(22) Anmeldetag: 08.06.2009
(51) Int. Cl.: D01H 13/22, D01H 13/26, B65H 61/00, D04B 9/14, G01N 27/22, G01N 27/24, G01N 33/36

(54) **KAPAZITIV ARBEITENDE SENSOREINHEIT ZUR ÜBERWACHUNG DER QUALITÄT VON FASERMATERIAL UND DAMIT AUSGERÜSTETE MASCHINE ZUR HERSTELLUNG VON MASCHENWARE**
CAPACITIVE ACTION SENSOR UNIT FOR MONITORING THE QUALITY OF FIBRE MATERIAL AND MACHINE FOR PRODUCING KNITTED GOODS PROVIDED THEREWITH
UNITÉ DE DÉTECTION À FONCTIONNEMENT CAPACITIF UTILISÉE POUR SURVEILLER LA QUALITÉ DE MATÉRIAUX FIBREUX ET MACHINE ÉQUIPÉE D'UNE TELLE UNITÉ SERVANT À PRODUIRE DES TISSUS MAILLÉS

(30) Priorität: 02.07.2008 DE 102008031130
(43) Veröffentlichungstag der Anmeldung: 06.04.2011
(73) Patentinhaber: SIPRA Patententwicklungs- und Beteiligungsgesellschaft mbH, 72461 Albstadt (DE)
(72) Erfinder: BAUER, Wolfgang, 70806 Kornwestheim (DE); FLAD, Axel, 72393 Burladingen (DE); ABT-SEITEL, Christine, 72458 Albstadt (DE); HAISCH, Erwin, 78554 Aldingen (DE); BISCHLER, Eduard, 68623 Lampertheim (DE); SCHWAB, Manuel, 72127 Kusterdingen (DE)
(74) Vertreter: Kohler Schmid Möbus
(86) Internationale Anmeldenummer: PCT/DE2009/000806
(87) Internationale Veröffentlichungsnummer: WO 2010/000218

(56) Entgegenhaltungen:
- EP-A- 1 035 413
- WO-A-2006/105676
- WO-A-2008/128363
- WO-A-2009/071062
- DE-B- 1 215 943
- US-A- 3 754 172

## Beschreibung

Die Erfindung betrifft eine Sensoreinheit der im Oberbegriff des Anspruchs 1 angegebenen Gattung und eine mit einer solchen Sensoreinheit ausgerüstete Maschine zur Herstellung von Maschenware.

In der Textilindustrie und hier insbesondere in der Spinnereitechnik ist es allgemein üblich, das zu verarbeitende Fasermaterial laufend auf seine Qualität zu überwachen. Unter "Fasermaterial" werden im Rahmen der vorliegenden Erfindung im Wesentlichen lineare, lang gestreckte, textile Materialien, insbesondere Faserbänder, Garne, Lunten, Flyerlunten, Filamente usw. verstanden, die einem bestimmten Verarbeitungsprozess unterworfen und zu diesem Zweck einem Streckwerk, einer Spinnvorrichtung od. dgl. zugeführt werden sollen.

Die Qualitätsüberwachung derartiger Fasermaterialien kann z. B. mit mechanischen, optischen oder kapazitiven Mitteln erfolgen. Für eine mechanische Überwachung werden Sensoreinheiten verwendet, die eine Rolle mit einer zur Aufnahme des Fasermaterials bestimmten Umfangsnut aufweisen, in die z. B. eine Tastrolle (DE 28 50 775 A1) oder ein Tastfinger (DE 199 50 901 A1) eingreift. Schwankungen in der Dichte oder Masse des Fasermaterials haben entsprechende Schwankungen der Lage der Tastrolle oder des Tastfingers zur Folge und werden mit diesen zugeordneten Wegmessern erfasst. Optische Sensoreinheiten (z. B. DE 32 37 371 A1) weisen aus Lumineszenzdioden und Phototransistoren gebildete Photozellen auf, die vom Fasermaterial durchlaufen werden. Kapazitiv arbeitende Sensoreinheiten schließlich sind mit einem Messkondensator versehen (z. B. EP 0 924 513 A1, DE 199 08 236 A1), der zwei parallel angeordnete Kondensatorplatten aufweist, die einen vom Fasermaterial durchlaufenen Durchgang begrenzen. Als Messgröße dient hier die Kapazität des Messkondensators, die sich entsprechend den Schwankungen im Fasermaterial verändert.

Da die Anwendung von Plattenkondensatoren in der Regel nicht ausreichend genau ist und z. B. durch unvermeidbares Signalrauschen gestört wird, sind auch bereits kapazitiv arbeitende Sensoreinheiten der eingangs bezeichneten Gattung bekannt geworden (z. B. PCT WO 2006/105676 A1), bei denen eine der beiden Kondensatorplatten an ihren Rändern mit zwei isoliert angebrachten Schutzelektroden versehen ist, an die eine sich zeitlich verändernde Spannung angelegt wird.

Sensoreinheiten der beschriebenen Art werden neuerdings auch für so genannte Spinnstrickmaschinen (z. B. PCT WO 2004/079068 A2) und andere maschenbildende Maschinen benötigt, denen anstelle eines üblichen Garns unmittelbar ein in einem Streckwerk oder auf andere Weise verfeinertes Fasermaterial in Form eines Faserbandes, einer Flyerlunte od. dgl. zugeführt wird. Mechanische Sensoreinheiten könnten hier mit Vorteil eingesetzt werden, da sie ein vergleichsweise geringes Signalrauschen verursachen, weitgehend unempfindlich gegen äußere Einflüsse sind und Schwankungen in der Fasermenge (z. B. Zahl der Fasern im Querschnitt eines Fasermaterials) mit ausreichender Genauigkeit abbilden. Ein Nachteil besteht allerdings darin, dass sie aus wenigstens zwei Komponenten, nämlich einem beweglichen Bauteil (Tastrolle, Tastfinger od. dgl.) und einem elektrischen Wegmesser bestehen. Da moderne Strickmaschinen z. B. 96 Stricksysteme aufweisen können und pro Stricksystem wenigstens je eine Sensoreinheit benötigt wird, wären die allein für die Überwachung des Fasermaterials aufzuwendenden Kosten unvertretbar hoch. Optische Sensoreinheiten sind für die Zwecke einer permanenten Überwachung von Fasermaterial an Spinnstrickmaschinen weitgehend ungeeignet, da sie einerseits zu ungenau arbeiten und andererseits empfindlich gegenüber unvermeidbaren Verunreinigungen sind.

Kapazitiv arbeitende Sensoreinheiten der eingangs bezeichneten Gattung haben schließlich zwar den Vorteil, dass sie preisgünstig herstellbar sind. Sie besitzen jedoch den Nachteil, dass sie über keine mechanische Führung für das Fasermaterial verfügen und die Messsignale eine zu geringe, kaum über ein Grundrauschen hinaus gehende Signalstärke haben, so dass nicht tolerierbare Schwankungen der Faserdichte oder Fasermasse nur ungenau ermittelt werden können. Das gilt zumindest für ihre Anwendung in Maschinen zur Herstellung von Maschenware. Da die Faserdicke und/oder die auf eine Einheitslänge bezogene Fasermasse der auf den Markt angebotenen Fasermaterialien - in deren Längs- bzw. Transportrichtung betrachtet - vergleichsweise häufigen Schwankungen unterworfen sind, müssen bisher entweder Maschenwaren mit ungleichförmiger Qualität, die durch Dick- oder Dünnstellen im Fasermaterial verursacht wird, in Kauf genommen oder aufwendige Maßnahmen getroffen werden, um derartige Schwankungen im Fasermaterial zu vermeiden oder die mit Fehlstellen versehenen Bereiche aus der Maschenware zu entfernen.

Ausgehend davon liegt der Erfindung das technische Problem zugrunde, die eingangs bezeichnete Sensoreinheit und eine damit ausgerüstete Maschine so auszubilden, dass sie zu vergleichsweise starken Messsignalen führen, gegen äußere Störungen weitgehend unempfindlich sind und dennoch preisgünstig hergestellt werden können.

Gelöst wird dieses Problem durch eine Sensoreinheit mit den kennzeichnenden Merkmalen des Anspruchs 1 und eine Maschine mit den kennzeichnenden Merkmalen des Anspruchs 25.

Die erfindungsgemäße Sensoreinheit ist mit einer Elektrodenanordnung versehen, die sich insbesondere für die Anwendung des so genannten kapazitiven 3-Elektroden-Messprinzips eignet. Messungen haben gezeigt, dass mit einer derartiger Elektrodenanordnung selbst dann, wenn sehr kleine kapazitive Änderungen vorhanden sind, vergleichsweise hohe Messsignalpegel erhalten werden. Aufgrund der erfindungsgemäßen Gestaltung der Messelektrode wird außerdem eine so gleichmäßige Zwangsführung des Fasermaterials erzielt, dass das vom Fasermaterial verursachte, unvermeidbare Signalrauschen nicht durch unkontrollierte Bewegungen das Fasermaterials im Messbereich vergrößert wird. Dadurch lassen sich durch Fasermasseschwankungen, Fremdkörpereinschlüsse od. dgl. verursachte Dick- oder Dünnstellen im Fasermaterial gut erkennen.

Weitere vorteilhafte Merkmale der Erfindung ergeben sich aus den Unteransprüchen.

Die Erfindung wird nachfolgend in Verbindung mit den beiliegenden Zeichnungen an Ausführungsbeispielen näher erläutert. Es zeigen:
Fig. 1 eine schematische Seitenansicht einer erfindungsgemäßen Sensoreinheit;
Fig. 2 einen Schnitt durch Sensoreinheit längs der Linie II-II der Fig. 1;
Fig. 3 eine perspektivische Darstellung eines praktischen Ausführungsbeispiels der erfindungsgemäßen Sensoreinheit;
Fig. 4 und 5 je einen Vertikalschnitt durch die Sensoreinheit nach Fig. 3 analog zu Fig. 1 und 2;
Fig. 6 eine vergrößerte Einzelheit X der Fig. 4;
Fig. 7 eine Rundstrickmaschine mit einer erfindungsgemäßen Sensoreinheit; und
Fig. 8 ein weiteres Ausführungsbeispiel der Sensoreinheit in einer der Fig. 5 entsprechenden Ansicht.

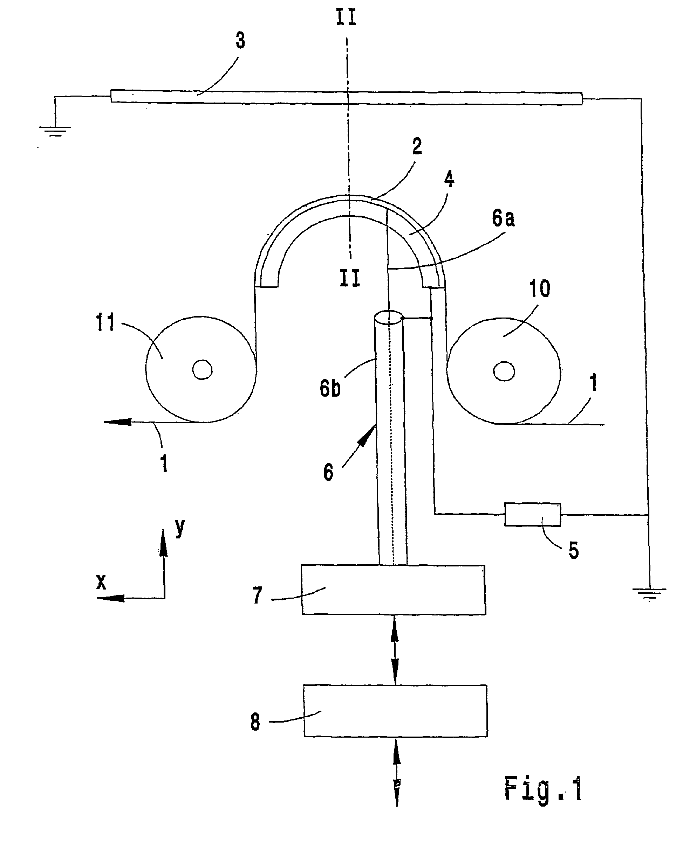

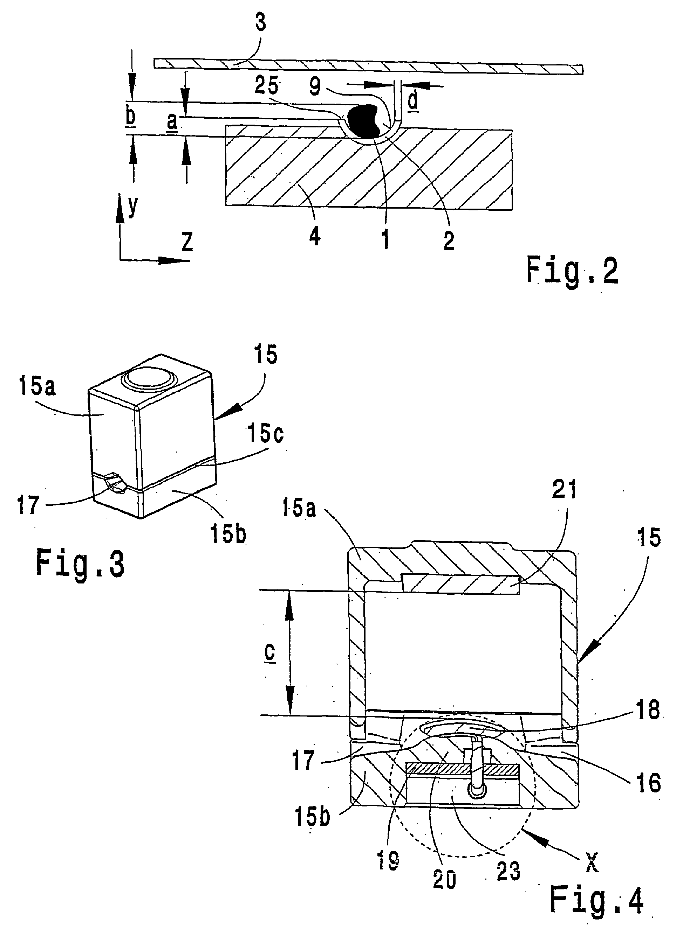

Fig. 1 und 2 zeigen eine erfindungsgemäße, kapazitiv arbeitende Sensoreinheit, die zur Überwachung der Qualität eines als Faserband, Flyerlunte od. dgl. vorliegendes Fasermaterial 1 dient, das in einem kontinuierlichen Prozess z. B. einem nicht dargestellten Streckwerk zugeführt wird. Das Fasermaterial 1 soll z. B. auf seine Homogenität und in diesem Zusammenhang insbesondere auf das Vorhandensein von Dick- oder Dünnstellen überprüft werden, die z. B. durch nicht gleichförmige Fasermassen pro Einheitslänge, eine unterschiedliche Anzahl von Fasern im Querschnitt des Fasermaterials 1 oder auch durch Fremdkörpereinschlüsse im Fasermaterial od. dgl. verursacht sein können. Ungleichmäßigkeiten dieser Art werden nachfolgend allgemein als "Fehlstellen" im Fasermaterial bezeichnet.

Die erfindungsgemäße Sensoreinheit arbeitet im Gegensatz zu bekannten Sensoreinheiten nach dem kapazitiven 3-Elektroden-Messprinzip. Sie enthält zu diesem Zweck eine Elektrodenanordnung, die eine erste, als Messelektrode 2 ausgebildete Elektrode, eine nachfolgend als Sendeelektrode 3 bezeichnete, zweite Elektrode und eine nachfolgend als Schirmelektrode 4 bezeichnete, dritte Elektrode aufweist. Alle drei Elektroden bestehen aus einem elektrisch gut leitenden Material, insbesondere Metall. Wie in Fig. 1 schematisch angedeutet ist, ist die Messelektrode 2 zwischen der Sendeelektrode 3 und der Schirmelektrode 4 angeordnet, die als Kondensatorelektroden aufgefasst werden können. Die Sendeelektrode 3 ist mit dem Signalausgang eines eine Wechselspannung von z. B. ca. 20 kHz liefernden Generators 5 verbunden, dessen anderer. Anschluss an die Schirmelektrode 4 angeschlossen ist und auf einem virtuellen Nullpotential liegt, das von der Auswerteelektronik gebildet wird. Zwischen der Messelektrode 2 und der Sendeelektrode 3 befindet sich ein Durchgang für das Fasermaterial 1. Außerdem ist die Messelektrode 2 mit dem Innenleiter 6a eines Koaxialkabels 6 verbunden, dessen Außenleiter 6b mit der Schirmelektrode 4 verbunden ist und daher auf demselben Potential wie diese liegt. Der Innenleiter 6a ist an einen Verstärker 7 angeschlossen, dessen Ausgang mit einer Auswerteelektronik 8 verbunden ist. Die Sendeelektrode 3 wird zusätzlich mit dem als Maschinenmasse bezeichneten Potential verbunden, das z. B. dem Massenpotential einer Rundstrickmaschine, eines streckwerks od. dgl. entspricht, denen das zu untersuchende Fasermaterial 1 zugeführt wird.

Eine für die Zwecke der Erfindung als besonders wesentlich erachtete Voraussetzung zur Erzielung sicherer Messergebnisse ist eine gleichmäßige Führung des Fasermaterials 1 durch die Sensoreinheit. Erfindungsgemäß ist daher vorgesehen, der Messelektrode 2 die aus Fig. 1 und 2 ersichtliche Form zu geben. Wird der Einfachheit halber angenommen, dass das Fasermaterial 1 in Richtung der X-Achse eines gedachten Koordinatensystems bewegt wird, die Elektroden 2, 3 und 4 entsprechend Fig. 1 in Richtung der Y-Achse des gedachten Koordinatensystems beabstandet sind und dessen Z-Achse in Fig. 1 senkrecht zur Zeichenebene verläuft, dann ist die Messelektrode 2 zunächst so gestaltet, dass sie in der Transportrichtung X einen bogenförmigen, z. B. etwa halbkreisförmigen Verlauf hat und mit ihrer Längsachse parallel zur XY-Ebene angeordnet ist. Außerdem ist die Messelektrode 2 so angeordnet, dass ihr konvex nach außen gewölbtes Teil eine der Sendeelektrode 3 zugewandte Oberseite und ein konkav gekrümmtes Teil eine der Schirmelektrode 4 zugewandte Unterseite der Messelektrode 2 bildet, wie aus Fig. 1 und 2 klar ersichtlich ist. Quer zur bogenförmigen Krümmung, d. h. in der YZ-Ebene gemäß Fig. 2, besitzt die Messelektrode 2 dagegen einen muldenförmigen Verlauf. Eine dadurch gebildete, zur Sendeelektrode 3 hin offene Mulde bildet eine stationäre Gleitfläche 9, längs welcher das Fasermaterial 1 durch die Sensoreinheit gleitet. Vorzugsweise ist die Gleitfläche 9 im Wesentlichen halbzylindrisch ausgebildet, wobei ihr Radius zweckmäßig geringfügig größer als der Durchmesser des Fasermaterials 1 ist. Insgesamt hat die Messelektrode 2 daher das Aussehen einer zur Sendeelektrode 3 hin offenen Dachrinne (Fig. 2), die in ihrer Längsrichtung, d. h. in Transportrichtung X, zusätzlich in der aus Fig. 1 ersichtlichen Weise bogenförmig gekrümmt ist.

Durch die beschriebene Form der Gleitfläche 9 wird eine gute seitliche Führung des Fasermaterials 1 sichergestellt, das in Fig. 2 mit einem kreisförmigen Querschnitt dargestellt ist, aber auch andere Querschnitte besitzen kann.

Damit das Fasermaterial längs der gesamten Messelektrode 2 gleichförmig an der Gleitfläche 9 anliegt, ist an einer Eintrittsseite der Sensoreinheit ein Niederhalter 10 und an der Austrittsseite der Sensoreinheit ein Niederhalter 11 angeordnet. Die Niederhalter 10 und 11 sind bevorzugt als Umlenkelemente ausgebildet, deren Ausgangs- bzw. Eingangsflächen in den Verlängerungen der Messelektrode 2 liegen, damit das Fasermaterial 1 der Messelektrode 2 tangential zugeführt und auch tangential wieder von ihr entfernt wird, wie Fig. 1 zeigt. Dadurch wird einerseits das Fasermaterial 1 einseitig fest gegen die Gleitfläche 9 gedrückt, während andererseits vermieden wird, dass das Fasermaterial 1 während des Durchgangs durch die Sensoreinheit zu stark umgelenkt oder geknickt wird. Störungen der auch vom Abstand des Fasermaterials 1 von der Gleitfläche 9 abhängigen Messsignale aufgrund einer ungleichförmigen Führung oder Anlage des Fasermaterials 1 auf der Gleitfläche 9 werden dadurch sicher vermieden.

Da erfahrungsgemäß die in Transportrichtung X gemessenen Längen üblicher Fehlstellen nur ca. 10 mm bis 20 mm betragen, sollte die X-Richtung gemessene Bogenlänge der Messelektrode 2 einerseits wenigstens etwa genauso groß, andererseits aber auch nicht wesentlich größer sein und höchstens z. B. 40 mm betragen. Um auch kleinste Fehlstellen zu entdecken, wird das Verhältnis Fehlstellenlänge zur Messelektrodenlänge vorzugsweise zwischen 1 : 1 und 1 : 4 gewählt. Außerdem sollte die in Y-Richtung gemessene Tiefe a (Fig. 2) der muldenförmigen Gleitfläche 9 höchstens gleich dem Durchmesser b des Fasermaterials 1, vorzugsweise jedoch höchstens halb so groß wie dieser sein. Dadurch werden ein günstiger Verlauf der elektrischen Feldlinien im Fasermaterial 1 und ein vergleichsweise starkes Messsignal erhalten, ohne dass die seitliche Führung des Fasermaterials 1 verloren geht.

Die Gleitfläche 9 und/oder mit dem Fasermaterial in Berührung kommende Flächen der Niederhalter 10, 11 können aus einem Material mit geringem Gleitwiderstand bestehen oder bei Bedarf mit einem den Gleitwiderstand reduzierenden Material beschichtet sein. Vorzugsweise bestehen die Niederhalter 10, 11 aus fest stehenden Umlenkelementen mit einem niedrigen Reibwert. Alternativ können die Niederhalter 10, 11 zur Verbesserung des Transports des Fasermaterials 1 aber auch als frei drehbar gelagerte Rollen ausgebildet sein oder aus angetriebenen Rollen bestehen und mit nicht dargestellten Antriebsmitteln verbunden sein, um sie während des Betriebs mit gleichen oder unterschiedlichen Umfangsgeschwindigkeiten in Umdrehungen zu versetzen.

Die Sendeelektrode 3 ist beispielsweise als ebene Metallplatte ausgebildet. Ihre Größe sollte so gewählt sein, dass ihre Parallel-Projektion auf die XZ-Ebene zu einer Fläche führt, die wenigstens genau so groß wie die durch eine entsprechende Parallel-Projektion der Messelektrode 2 erhaltene Fläche ist und diese überdeckt. Diese Maßnahme dient dem Zweck, ein ausreichend ausgedehntes, elektrisches Feld zu erzeugen, das die gesamte Messelektrode 2 erfasst und den gesamten, auf dieser gleitenden Abschnitt des Fasermaterials 1 durchflutet.

Während die Sendeelektrode 3 der konvex gewölbten, nach oben offenen Oberseite der Messelektrode 2 gegenübersteht, ist die Schirmelektrode 4 der konkav gewölbten Unterseite der Messelektrode 2 zugewandt. Die Schirmelektrode 4 ist vorzugsweise so ausgebildet, dass sie die Messelektrode 2 umschließt, ausgenommen an der die Gleitfläche 9 aufweisenden Oberseite. Außerdem muss die Schirmelektrode 4 natürlich elektrisch von der Messelektrode 2 isoliert sein, was durch zwischen beiden angeordnete Isoliermittel sichergestellt wird. Insgesamt hat die Schirmelektrode 4 zweckmäßig eine wirksame, der Unterseite der Messelektrode 2 zugewandte Fläche, die wenigstens genau so groß wie die ihr zugewandte wirksame Fläche der Messelektrode 2 ist. Die Form der Schirmelektrode 4 wird vorzugsweise so gewählt, dass die zwischen ihr und der Sendeelektrode 3 verlaufenden Feldlinien möglichst auch die Messelektrode 2 durchsetzen und nicht seitlich an dieser vorbeilaufen.

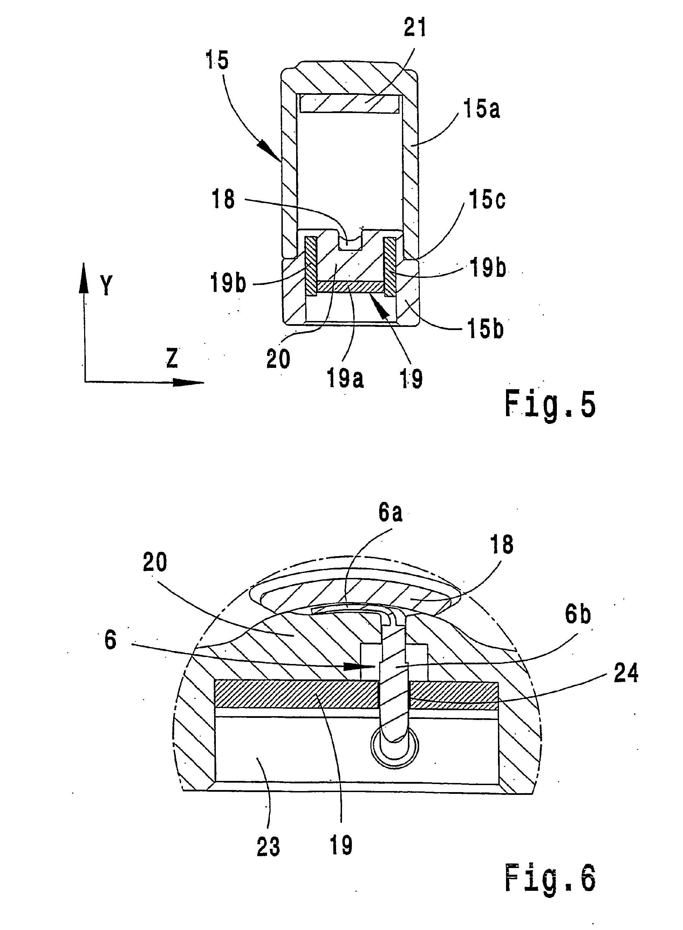

Fig. 3 bis 6 zeigen ein für die praktische Anwendung besonders gut und derzeit für am besten gehaltenes Ausführungsbeispiel der erfindungsgemäßen Sensoreinheit. Diese enthält ein im Wesentlichen geschlossenes Gehäuse 15, das ein Oberteil 15a und ein Unterteil 15b aufweist, die längs einer Trennebene 15c (Fig. 3 und 5) aneinander grenzen, die eine Eintrittsöffnung 16 und eine Austrittsöffnung 17 für das nicht dargestellte Fasermaterial 1 etwa in der Mitte durchsetzt (Fig. 3 und 4). Eine nach Art einer Dachrinne ausgebildete Messelektrode 18, die analog zu Fig. 1 und 2 nach oben offen und in X-Richtung nach oben konvex gewölbt ist, ist im Wesentlichen wie in Fig. 1 und 2 ausgebildet, im Unterschied dazu aber über einen Bogen von weniger als ca. 180° erstreckt. Die Enden der Messelektrode 18 sind vorzugsweise so angeordnet, dass sie tangential in die Eintritts- bzw. Austrittsöffnung 16 bzw. 17 münden (Fig. 4).

Wie insbesondere Fig. 5 zeigt, sind die Messelektrode 18 und eine ihrer Unterseite zugewandte Schirmelektrode 19 in einen vorzugsweise als Gießharzkörper ausgebildeten Isolator 20 aus einem elektrisch isolierenden Material eingebettet und dadurch einerseits zu einem einstückigen, das Unterteil 15b des Gehäuses 15 bildenden Körper miteinander verbunden, andererseits elektrisch voneinander isoliert. Ferner zeigt Fig. 5, dass die Schirmelektrode 19 U-förmig ausgebildet ist und einen Bodenabschnitt 19a aufweist, an dessen seitlichen Enden zwei in Y-Richtung ragende Seitenwände 19b vorgesehen sind. Die Anordnung ist insbesondere so getroffen, dass die Schirmelektrode 19 die Unterseite und die Seitenteile der Messelektrode 18 U-förmig umgibt und diese daher außer nach oben allseitig umschließt. Dadurch wird eine Konzentration der Feldlinien auf die Messelektrode 18 erreicht und durch Unregelmäßigkeiten der Lunten eine maximale Potentialveränderung an der Messelektrode 18 hervorgerufen.

Die Sensoreinheit nach Fig. 3 bis 6 weist außerdem eine der Oberseite der Messelektrode 18 zugewandte, plattenförmige Sendeelektrode 21 auf, die parallel zur XZ-Ebene angeordnet und an der Innenwand der Decke des topfförmig ausgebildeten Oberteils 15a befestigt ist. Die Sendeelektrode 21 steht der Messelektrode 18 unter Bildung eines Durchgangs für das Fasermaterial mit einem Abstand c (Fig. 4) gegenüber. Dieser Abstand c, vom höchsten Punkt der Messelektrode 18 aus gemessen, sollte einerseits höchstens dem Einbis Zweifachen des Durchmessers des zu überwachenden Fasermaterials 1 betragen, andererseits aber so groß sein, dass auch Verdickungen der Lunten frei passieren können.

Für den Fall, dass die Messelektrode 18 analog zu Fig. 1 und 2 über eine Bogenlänge von ca. 180° erstreckt wird, ist es zweckmäßig, den Niederhaltern 10 und 11 nach Fig. 1 entsprechende Niederhalter auch für die Sensoreinheit nach Fig. 3 bis 6 vorzusehen und diese auf einer gemeinsamen, z. B. durch den Isolator 20 gebildeten oder am Oberteil 15a vorgesehenen Grundplatte zu montieren. Es ist dann möglich, auch die Niederhalter in die vom Gehäuse 15 umschlossene Sensoreinheit zu integrieren.

Schließlich ist es vorteilhaft, das Oberteil 15a und das Unterteil 15b leicht lösbar miteinander zu verbinden, damit ein einfaches Auflegen des Fasermaterials 1 auf die Messelektrode 18 möglich ist. Besonders zweckmäßig ist es, das Oberteil 15a zu diesem Zweck schwenkbar am Unterteil 15b zu befestigen, damit das Gehäuse 15 im Bereich der Trennebene 15c aufgeklappt werden kann.

Im Übrigen ist vorgesehen, die komplett vorgefertigte Sensoreinheit direkt an einer Strickmaschine, einem Streckwerk od. dgl. zu befestigen und dadurch gleichzeitig mit der Maschinenmasse zu verbinden, wie in Fig. 1 schematisch angedeutet ist.

Fig. 4 und 6 zeigen schließlich den Anschluss des auch aus Fig. 1 ersichtlichen Koaxialkabels 6, das z. B. durch eine im Isolator 20 ausgebildete Aussparung 23 hindurch in ein Loch der Schirmelektrode 19 eingesetzt wird. An dieser Stelle kann der Außenleiter 6b durch eine Lötverbindung 24 elektrisch mit der Schirmelektrode 19 verbunden werden. Der Innenleiter 6a wird dagegen z. B. an die Unterseite der Messelektrode 18 angelegt und durch ein leitfähiges Gießharz od. dgl. an dieser fixiert.

Die beschriebene Ausbildung der Sensoreinheit bringt den Vorteil mit sich, dass sie lediglich an einem auf Maschinenmasse liegenden Bauteil befestigt werden braucht und nach dem Anlegen der Generatorspannung zwischen die Schirmelektrode 4, 19 und die Sendeelektrode 3, 21 voll funktionsfähig ist, ohne dass komplizierte Einstellarbeiten erforderlich sind.

Zur Inbetriebnahme der Sensorseinheit nach dem 3-Elektroden-Messprinzip wird mittels des Generators 5 ein Anregungsfeld zwischen der Sendeelektrode 3, 21 (Maschinenmasse) und der Schirmelektrode 4, 19 eingespeist, wobei die Messelektrode 2, 18 zwischen diesen beiden eingeprägten Potentialen angeordnet und über das Fasermaterial 1 an das elektrische Feld angekoppelt ist. Die Messelektrode 2, 18 befindet sich daher auf einem schwimmenden Potential, wobei ihr Abstand zur Schirmelektrode 4, 19 vorzugsweise konstant und kleiner als der Abstand c (Fig. 4) ist. Das über die Messelektrode 2, 18 gleitende Fasermaterial 1 bewirkt daher beim Auftreten von Inhomogenitäten (Fehlstellen) eine Veränderung des elektrischen Feldes und damit auch eine Potentialänderung an der Messelektrode 2 und 18. Durch die beschriebene Gestaltung der Sendeelektrode 3, 21 und der Schirmelektrode 4, 19 und weitere Optimierungen der Sensorgeometrie kann die erzielbare Signalstärke des Messsignals weiter verbessert werden. Dies gelingt insbesondere dann, wenn das vom Generator 5 erzeugte Anregungsfeld (bzw. der Feldlinienverlauf) entsprechend der obigen Beschreibung auf die Messelektrode 2, 18 konzentriert und für eine intensive Durchflutung des Fasermaterials 1 gesorgt wird. Mit anderen Worten sollte der Feldanteil, der nicht das Fasermaterial 1 durchflutet, möglichst klein oder zumindest konstant gehalten werden. In der erfindungsgemäßen Anordnung wird bewirkt, dass im Randbereich der Messelektrode 2, 18 relativ konstante Bedingungen herrschen und Wärmebewegungen, Temperaturdriften der Isolationsmaterialien, insbesondere deren Änderungen der Dielektrizitätskonstante, nicht zu Potentialänderungen der Messelektrode 2, 18 führen. Zu diesem Zweck ist es bei dem anhand der Fig. 1 und 2 beschriebenen Ausführungsbeispiel auch besonders vorteilhaft, eine Wandstärke d der Messelektrode 2, 18 insbesondere in den an die Gleitfläche 9 grenzenden, in Fig. 2 durch das Bezugszeichen 25 gekennzeichneten Bereichen nicht größer als ca. 5 mm zu wählen. Außerdem hat es sich als zweckmäßig erwiesen, den Abstand zwischen der Sendeelektrode 3, 21 und der Messelektrode 2, 18 möglichst klein zu wählen. Dadurch wird eine hohe Feldstärke des Anregungsfeldes bewirkt, die unmittelbar die Messsteilheit positiv beeinflusst.

Der Verstärker 7 und zumindest Teile der Auswerteelektronik 8 für die von der Messelektrode 2, 18 abgegebenen Messsignale können vorzugsweise ebenfalls in der Sensoreinheit bzw. im Gehäuse 15 untergebracht werden. In diesem Fall wird das Messsignal z.B. mit Hilfe von Verstärkern auf einen solchen Signalpegel angehoben, dass es anschließend unter Verzicht auf Koaxialkabel frei von Störbeeinflussungen mit einfachen Kabeln zur Auswerteelektronik 8 weitergeleitet werden kann.

Die Auswertung der Messsignale kann in an sich beliebiger Weise erfolgen, wird bevorzugt aber mit Hilfe von Schaltungsanordnungen durchgeführt, die speziell für das kapazitive 3-Elektroden-Messprinzip entwickelt wurden und zur Detektion extrem kleiner Kapazitätsänderungen geeignet sind. Insbesondere wird in diesem Zusammenhang auf eine Auswertetechnik verwiesen, die aus der Druckschrift DE 100 27 507 C1 bekannt ist, welche zur Vermeidung von Wiederholungen hiermit durch Referenz auf sie zum Gegenstand der vorliegenden Offenbarung gemacht wird.

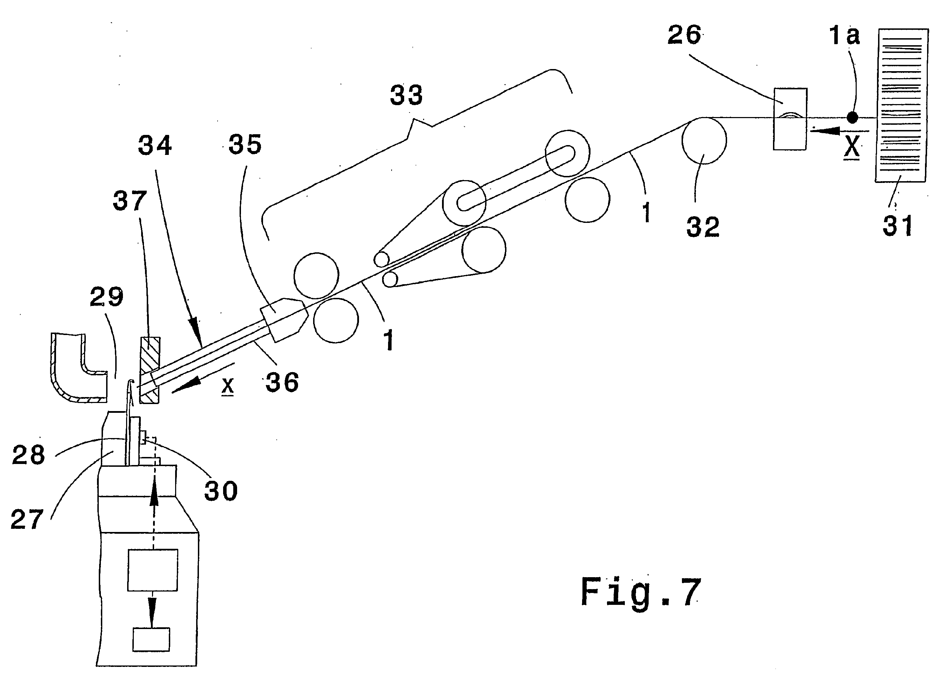

Fig. 7 zeigt eine Teilansicht einer Maschine zur Herstellung von Maschenware am Beispiel einer Rundstrickmaschine mit einer Vielzahl von Maschenbildungsstellen (Stricksystemen), denen wenigstens je eine der beschriebenen, hier mit dem Bezugszeichen 26 bezeichneten Sensoreinheit zugeordnet ist.

Die Rundstrickmaschine enthält einen Nadelzylinder 27, in dem übliche Stricknadeln 28 verschiebbar gelagert sind, die an einer nachfolgend als Stricksystem 29 bezeichneten Maschenbildungsstelle mit Hilfe von nicht näher dargestellten Schlossteilen 30 in eine zur Aufnahme des Fasermaterials 1 geeignete Faseraufnahmestellung bewegt werden können. Das Fasermaterial 1 wird der Rundstrickmaschine von Vorratsbehältern 31 wie z. B. Kannen, Vorratsspulen od. dgl. aus zugeführt.

Das Fasermaterial 1 wird über ein nicht dargestelltes Transportmittel und ggf. eine Umlenkrolle 32 einem Streckwerk 33 zugeführt. Jedem der Stricksysteme 29, von denen in Fig. 7 nur eines dargestellt ist, ist ein derartiges Streckwerk 33 zugeordnet, das in an sich bekannter Weise z. B. drei oder mehr Paare von Streckwalzen aufweist.

Das aus dem Streckwerk 33 kommende Fasermaterial 1 wird in bekannter Weise vorzugsweise mit Hilfe einer allgemein mit dem Bezugszeichen 34 bezeichneten Spinn- bzw. Transportvorrichtung einem zugeordneten Stricksystem 29 zugeführt. Die Transportvorrichtung 34 enthält z. B. ein Drallorgan 35 und ein an dieses angeschlossenes Spinn- bzw. Transportrohr 36, das an einem Fadenführer 37 endet, der wie üblich dicht vor den Stricknadeln 28 und so angeordnet ist, dass das Fasermaterial 1 in die Haken der Stricknadeln 28 eingelegt wird.

Rundstrickmaschinen der beschriebenen Art sind z. B. aus der Druckschrift PCT WO 2004/079068 bekannt, die hiermit zur Vermeidung von Wiederholungen durch Referenz auf sie zum Gegenstand der vorliegenden Offenbarung gemacht wird.

Ziel der Erfindung ist es, das Auftreten der eingangs erwähnten Fehlstellen in der fertigen Maschenware zu verhindern. Zu diesem Zweck wird erfindungsgemäß zu- nächst ganz allgemein vorgeschlagen, das Fasermaterial 1 vor seiner Ankunft an einem Stricksystem 29 auf die erforderlichen Qualitätsmerkmale, insbesondere Dicke- und Masseschwankungen zu überprüfen, und beim Erkennen von Fasermaterialabschnitten, die sich durch unzulässige Abweichungen von einer vorgewählten Qualität auszeichnen, zu verhindern, dass diese fehlerhaften Fasermaterialabschnitte in die Stricknadeln 28 eingelegt werden. Dies wird dadurch erreicht, dass das Fasermaterial 1 mit Hilfe der entsprechend Fig. 1 bis 6 ausgebildeten Sensoreinheit 26 überwacht und beim Erkennen einer Fehlstelle, die in Fig. 7 schematisch durch einen Punkt 1a angedeutet ist, der Maschenbildungsprozess unterbrochen, die Fehlstelle 1a aus dem Fasermaterial 1 entfernt und der Maschenbildungsprozess dann fortgesetzt wird. Die Unterbrechung des Maschenbildungsprozesses kann z. B. dadurch erfolgen, dass die Strickwerkzeuge 28 am betreffenden Stricksystem 29 ab Erkennung einer Fehlstelle 1a ohne Abwerfen zuvor gebildeter Maschen und ohne Aufnahme von Fasermaterial 1 am Stricksystem 29 vorbeigeführt und zu diesem Zweck z. B. in eine Rundlaufbahn gesteuert werden. Die Sensoreinheit 26 kann entsprechend Fig. 7 in Transportrichtung x vor dem Streckwerk 33 oder auch an einer anderen, vor dem betreffenden Stricksystem 29 liegenden Stelle angeordnet sein.

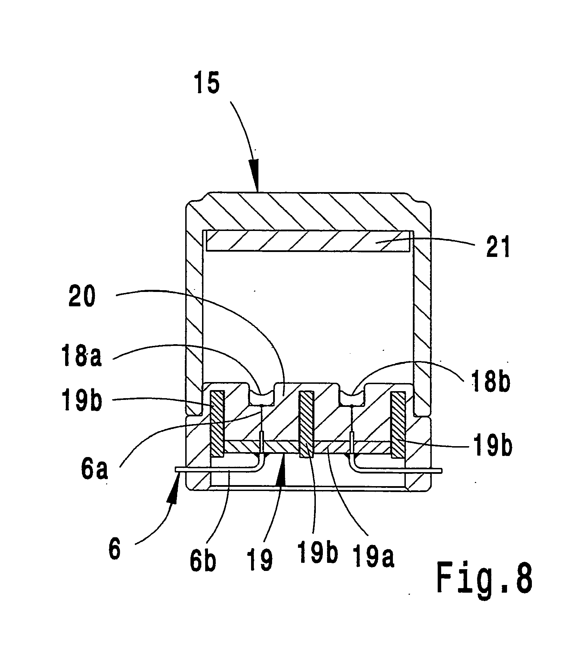

In einem weiteren Ausführungsbeispiel, das in Fig. 8 dargestellt ist, werden in dem zwischen der Sendeelektrode 21 und der Schirmelektrode 19 bestehenden Anregungsfeld mehrere, z. B. entsprechend Fig. 3 bis 6 ausgebildete Meßelektroden 18a, 18b nebeneinander und isoliert voneinander angeordnet. Damit können in einer Sensoreinheit mehrere, parallel zueinander geführte Fasermaterialien 1 gleichzeitig auf Fehlstellen überwacht werden. Die Sendeelektrode 21 und die Schirmelektrode 19 können in der aus Fig. 3 bis 6 ersichtlichen Form mehrfach verwendet und im Gehäuse 15 zu einer Baueinheit zusammengefaßt werden, wobei sie lediglich durch den Isolator 20 oder andere geeignete Isoliermittel elektrisch voneinander getrennt werden müssen.

Besonders vorteilhaft ist es, die Sendeelektrode 21 in diesem Ausführungsbeispiel als eine einzige, große Elektrode auszuführen, die alle vorhandenen Meßelektroden 18a, 18b überdeckt. Entsprechend kann die Schirmelektrode 19 dadurch ergänzt werden, daß ihr Bodenabschnitt 19a entsprechend verlängert und eine weitere von ihr aufragende Seitenwand 19b vorgesehen wird. Auf diese Weise wird gemäß Fig. 8 eine mehr w- oder kammförmige Schirmelektrode 19 erhalten, wobei die Seitenwände 19b paarweise je eine Meßelektrode 18a, 18b seitlich abschirmen.

Aus der Zusammenfassung der Schirmelektrode 19 gemäß Fig. 8 resultiert außerdem der Vorteil, dass auf dieser direkt die Verstärker 7 (Fig. 1) für die einzelnen Meßelektroden 18a, 18b angebracht werden können, wodurch ein kompakter, integrierter Schaltungsaufbau erhalten wird. Schließlich ist es beim Ausführungsbeispiel nach Fig. 8 in vorteilhafter Weise nur erforderlich, den Außenleiter 6b eines der zu den Messelektroden 18a, 18b führenden Koaxialkabels 6 an das Schirmpotential anzuschließen und mit der Schirmelektrode 19 z. B. durch Schweißpunkte zu verbinden, da die Außenleiter 6b aller Koaxialkabel 6 in der Auswerteeinheit 8 ohnehin auf ein gemeinsames Potential gelegt werden.

Die Erfindung ist nicht auf die beschriebenen Ausführungsbeispiele beschränkt, die auf vielfache Weise abgewandelt werden können. Das gilt insbesondere für die oben angegebenen Maße für die verschiedenen Krümmungsradien, Abstände und sonstigen Abmessungen, da diese Maße u. a. auch von der Art und den Dimensionen des untersuchten Fasermaterials 1 abhängen. Aus demselben Grund kann es zweckmäßig sein, der Gleitfläche 9 einen mehr kreisförmigen Querschnitt (Fig. 2) oder einen mehr quadratischen oder rechteckigen Querschnitt zugeben. Weiter wäre es möglich, den Sendeelektroden 3, 21 einen an die Krümmung der Messelektroden 2, 18 angepassten bogenförmigen Verlauf zu geben, um dadurch die Feldlinien homogen auf die Messelektroden 2, 18 zu konzentrieren. Außerdem ist klar, dass die Meßelektroden 2, 18 bei der Verwendung von Lunten mit leitfähigen Anteilen, z. B. in Form von Metallfäden, eine dünne Isolationsschicht aufweisen sollten, um elektrische Kurzschlüsse zu vermeiden. Ferner ist klar, dass die Erfindung nicht nur Maschinen zur Herstellung von Maschenware umfasst, die entsprechend Fig. 7 ausgebildet sind, sondern auch z. B. solche, bei denen die Verfeinerung bzw. Verstreckung des Fasermaterials nicht mit Hilfe von Streckwerken, sondern auf andere Weise erfolgt (z. B. PL 350 489 A).

## Patentansprüche

1. Kapazitiv arbeitende Sensoreinheit zur Überwachung der Qualität von in einer Transportrichtung bewegtem Fasermaterial (1), enthaltend eine Elektrodenanordnung, die eine erste, wenigstens eine zweite und eine dritte Elektrode (2, 18; 3, 21; 4, 19) und einen von der ersten und der zweiten Elektrode begrenzten Durchgang für das Fasermaterial (1) aufweist, wobei die erste Elektrode eine Messelektrode (2, 18) mit einer Oberseite ist, die eine in Transportrichtung erstreckte Gleitfläche (9) für das Fasermaterial (1) enthält, dass die zweite Elektrode eine der Oberseite der Messelektrode (2, 18) mit Abstand gegenüberstehende Sendeelektrode (3, 21) ist und dass die dritte Elektrode eine Schirmelektrode (4, 19) ist, **dadurch gekennzeichnet, dass** die Messelektrode (2, 18) eine von der Schirmelektrode (4, 19) umgebene Unterseite besitzt.

2. Sensoreinheit nach Anspruch 1, **dadurch gekennzeichnet, dass** die Messelektrode (2, 18) in Transportrichtung bogenförmig gekrümmt ist und eine konvexe Oberseite und eine konkave Unterseite aufweist

3. Sensoreinheit nach Anspruch 1 oder 2, **dadurch gekennzeichnet, dass** die Gleitfläche (9) muldenförmig ausgebildet ist.

4. Sensoreinheit nach einem der Ansprüche 1 bis 3, **dadurch gekennzeichnet, dass** einer Eintritts- und einer Austrittsseite der Messelektrode (2) je ein zur Einwirkung auf das Fasermaterial (1) bestimmter, eine gleichförmige flächige Auflage des Fasermaterials (1) auf der Gleitfläche (9) erzwingender Niederhalter (10, 11) zugeordnet ist.

5. Sensoreinheit nach einem der Ansprüche 1 bis 4, **dadurch gekennzeichnet, dass** der Radius der muldenförmigen Gleitfläche (9) geringfügig größer ist, als dem Radius des Fasermaterials (1) entspricht.

6. Sensoreinheit nach einem der Ansprüche 1 bis 5, **dadurch gekennzeichnet, dass** die Messelektrode (18) und die Schirmelektrode (19) auf einander zugewandten Seiten in einen Isolator (20) eingebettet und dadurch elektrisch gegeneinander isoliert sind.

7. Sensoreinheit nach Anspruch 6, **dadurch gekennzeichnet, dass** ein den Isolator (20) enthaltendes Unterteil (15b) und ein auf dieses aufsetzbares, die Sendeelektrode (21) aufnehmendes Oberteil (15a) ein Sensorgehäuse (15) bilden, das an der Eintritts- und Austrittsseite der Messelektrode (18) je eine Eintritts- bzw. Austrittsöffnung (16, 17) für das Fasermaterial (1) aufweist.

8. Sensoreinheit nach Anspruch 7, **dadurch gekennzeichnet, dass** das Unterteil (15b) und das Oberteil (15a) lösbar oder schwenkbar miteinander verbunden sind.

9. Sensoreinheit nach Anspruch 7 oder 8, **dadurch gekennzeichnet, dass** im Sensorgehäuse (15) Mittel zur Umwandlung von von der Messelektrode (2, 18) abgegebenen Messsignalen in Signalpegel untergebracht sind, die eine Weiterleitung an eine Auswerteelektronik (8) über normale Kabel ermöglichen.

10. Sensoreinheit nach einem der Ansprüche 1 bis 9, **dadurch gekennzeichnet, dass** die in Transportrichtung gemessene Länge der Gleitfläche (9) und die Länge von zu ermittelnden Fehlstellen im Fasermaterial (1) in einem vorgewählten Verhältnis zueinander stehen, das vorzugsweise 1:1 bis 4:1 beträgt.

11. Sensoreinheit nach einem der Ansprüche 1 bis 10, **dadurch gekennzeichnet, dass** ein Abstand (c) der Sendeelektrode (21) von einem höchsten Punkt der Messelektrode (18) eine Größe besitzt, die einem Ein- bis Zweifachen des Durchmessers des Fasermaterials (1) entspricht.

12. Sensoreinheit nach einem der Ansprüche 1 bis 11, **dadurch gekennzeichnet, dass** an die Gleitfläche (9) grenzende Randbereiche (25) der Messelektrode (2) eine Breite von nicht mehr als 5 mm besitzen.

13. Sensoreinheit nach einem der Ansprüche 1 bis 12, **dadurch gekennzeichnet, dass** eine Tiefe (a) der Gleitfläche (9) höchsten gleich dem Durchmesser des Fasermaterials (1) ist.

14. Sensoreinheit nach einem der Ansprüche 1 bis 13, **dadurch gekennzeichnet, dass** eine durch Parallel-Projektion der Sendeelektrode (3, 21) erhaltene Fläche wenigstens genau so groß wie eine durch Parallel-Projektion der Messelektrode (2, 18) erhaltene Fläche ist und diese überdeckt.

15. Sensoreinheit nach einem der Ansprüche 1 bis 14, **dadurch gekennzeichnet, dass** die Schirmelektrode (4, 19) eine wenigstens genau so große wirksame Fläche wie die Messelektrode (2, 18) besitzt.

16. Maschine zur Herstellung von Maschenware mit wenigstens einer Maschenbildungsstelle (29), Mitteln (33, 34) zur Zuführung von verstrecktem Fasermaterial (1) zur Maschenbildungsstelle (29) und einer Sensoreinheit (26) zur Überwachung der Qualität des Fasermaterials (1), **dadurch gekennzeichnet, dass** die Sensoreinheit (26) nach wenigstens einem der Ansprüche 1 bis 15 ausgebildet ist.

## Claims

1. Capacitive action sensor unit for monitoring the quality of fibre material (1) moving in a transport direction, including an electrode arrangement, which has a first, at least one second and one third electrode (2, 18; 3, 21; 4, 19) and a passage for the fibre material (1) delimited by the first and the second electrode, wherein the first electrode is a measurement electrode (2, 18) with an upper side, which contains a slide face (9) extended in transport direction for the fibre material (1), that the second electrode is a transmission electrode (3, 21) standing at a distance opposite the upper side of the measurement electrode (2, 18) and that the third electrode is a shield electrode (4, 19), **characterised in that** the measurement electrode (2, 18) has an underside surrounded by the shield electrode (4, 19).

2. Sensor unit according to claim 1, **characterised in that** the measurement electrode (2, 18) is curved in an arc shape in transport direction and has a convex upper side and a concave underside.

3. Sensor unit according to claim 1 or 2, **characterised in that** the slide face (9) is configured in a trough shape.

4. Sensor unit according to one of claims 1 to 3, **characterised in that** a hold-down element (10, 11), which is intended for action on the fibre material (1) and forces the fibre material (1) to lie uniformly flat on the slide face (9), is respectively associated with an inlet side and an outlet side of the measurement electrode (2).

5. Sensor unit according to one of claims 1 to 4, **characterised in that** the radius of the trough-shaped slide face (9) is sightly larger than the radius of the fibre material (1).

6. Sensor unit according to one of claims 1 to 5, **characterised in that** the measurement electrode (18) and the shield electrode (19) are embedded into an insulator (20) on facing sides and are thus electrically insulated from one another.

7. Sensor unit according to claim 6, **characterised in that** a lower part (15b) containing the insulator (20) and an upper part (15a), which can be placed thereon and receives the transmission electrode (21 form a sensor housing (15), which respectively has an inlet or outlet (16, 17) for the fibre material (1) on the inlet side and outlet side of the measurement electrode (18).

8. Sensor unit according to claim 7, **characterised in that** the lower part (1, 5b) and the upper part (15a) are connected to one another to be releasable or able to pivot.

9. Sensor unit according to claim 7 or 8, **characterised in that** elements for transforming measurement signals emitted by the measurement electrode (2, 18) in the signal level are housed in the sensor housing (15) and enable forwarding to an electronic evaluation unit (8) via normal cables.

10. Sensor unit according to one of claims 1 to 9, **characterised in that** the length of the slide face (9) measured in transport direction and the length of faults to be determined in the fibre material (1) are in a preselected ratio to one another that preferably amounts to 1:1 to 4:1.

11. Sensor unit according to one of claims 1 to 10, **characterised in that** a spacing (c) of the transmission electrode (21) from a highest point of the measurement electrode (18) has a magnitude corresponding to once to twice the diameter of the fibre material (1).

12. Sensor unit according to one of claims 1 to 11, **characterised in that** edge regions (25) of the measurement electrode (2) adjoining the slide face (9) have a width of no more than 5 mm.

13. Sensor unit according to one of claims 1 to 12, **characterised in that** a depth (a) of the slide face (9) is at most equal to the diameter of the fibre material (1).

14. Sensor unit according to one of claims 1 to 13, **characterised in that** a surface obtained by parallel projection of the transmission electrode (3, 2,1) is at least exactly as large as a surface obtained by parallel projection of the measurement electrode (2, 18) and overlaps this.

15. Sensor unit according to one of claims 1 to 14, **characterised in that** the shield electrode (4, 19) has an active surface that is at least exactly the same size as the measurement electrode (2, 18).

16. Machine for producing knitted goods with at least one stitch-forming location (29), elements (33, 34) for feeding stretched fibre material (1) to the stitch-forming location (29) and a sensor unit (26) for monitoring the quality of the fibre material (1), **characterised in that** the sensor unit (26) is configured in accordance with at least one of claims 1 to 15.

## Revendications

1. Unité de détection à fonctionnement capacitif pour surveiller la qualité d'un matériau fibreux (1) déplacé dans une direction de transport, contenant un agencement d'électrodes qui présente une première, au moins une deuxième et une troisième électrode (2, 18 , 3, 21 ; 4, 19) et un passage pour le matériaux fibreux (1) limité par la première et la deuxième électrode, la première électrode étant une électrode de mesure (2, 18) avec une face supérieure qui contient une surface de glissement (9) qui s'étend dans la direction de transport pour le matériaux fibreux (1), la deuxième électrode étant une électrode émettrice (3, 21) qui fait face, à distance, à la face supérieure de l'électrode de mesure (2, 18) et la troisième électrode étant une électrode écran (4, 19), **caractérisée en ce que** l'électrode de mesure (2, 18) possède une face inférieure entourée par l'électrode écran (4, 19).

2. Unité de détection selon la revendication 1, **caractérisée en ce que** l'électrode de mesure (2, 18) est courbée en forme d'arc dans la direction de transport et présente une face supérieure convexe et une face inférieure concave.

3. Unité de détection selon la revendication 1 ou 2, **caractérisée en ce que** la surface de glissement (9) est réalisée en forme de cuvette.

4. Unité de détection selon une des revendications 1 à 3, **caractérisée en ce que** des rouleaux presseurs (10, 11) destinés à agir sur le matériau fibreux (1), qui imposent un appui plan uniforme du matériau fibreux (1) sur la surface de glissement (9), sont respectivement associés à un côté entrée et à un côté sortie de l'électrode de mesure (2).

5. Unité de détection selon une des revendications 1 à 4, **caractérisée en ce que** le rayon de la surface de glissement (9) en forme de cuvette est légèrement plus grand que le rayon du matériau fibreux (1).

6. Unité de détection selon une des revendications 1 à 5, **caractérisée en ce que** l'électrode de mesure (18) et l'électrode écran (19) sont noyées dans un isolant (20) sur des faces tournées l'une vers l'autre et de ce fait isolées l'une par rapport à l'autre.

7. Unité de détection selon la revendication 6, **caractérisée en ce qu'**une partie inférieure (15b) contenant l'isolant (20) et une partie supérieure (15a), pouvant être posée sur celle-ci et recevant l'électrode émettrice (21), forment un boîtier de capteur (15) qui présente une ouverture d'entrée, respectivement de sortie (16, 17), pour le matériau fibreux (1) du côté entrée, respectivement sortie, de l'électrode de mesure (18).

8. Unité de détection selon la revendication 7, **caractérisée en ce que** la partie inférieure (15b) et la partie supérieure (15a) sont reliées entre elles de manière détachable ou pivotante.

9. Unité de détection selon la revendication 7 ou 8, **caractérisée en ce que** des moyens pour convertir des signaux de mesure délivrés par l'électrode de mesure (18) en niveaux de signal, qui permettent une transmission à une électronique d'évaluation (8) via des câbles normaux, sont logés dans le boîtier de capteur (15).

10. Unité de détection selon une des revendications 1 à 9, **caractérisée en ce que** la longueur de la surface de glissement (9) mesurée dans la direction de transport et la longueur de défauts à déterminer dans le matériau fibreux (1) ont entre elles un rapport présélectionné qui est de préférence compris entre 1:1 et 4:1.

11. Unité de détection selon une des revendications 1 à 10, **caractérisée en ce qu'**une distance (c) de l'électrode émettrice (21) à un point culminant de l'électrode de mesure (18) possède une grandeur qui correspond à un à deux fois le diamètre du matériau fibreux (1).

12. Unité de détection selon une des revendications 1 à 11, **caractérisée en ce que** les zones de bord (25) de l'électrode de mesure (2) qui jouxtent la surface de glissement (9) possèdent une largeur qui n'est pas supérieure à 5 mm.

13. Unité de détection selon une des revendications 1 à 12, **caractérisée en ce qu'**une profondeur (a) de la surface de glissement (9) est au plus égale au diamètre du matériau fibreux (1).

14. Unité de détection selon une des revendications 1 à 13, **caractérisée en ce qu'**une surface obtenue par une projection parallèle de l'électrode émettrice (3, 21) est au moins exactement aussi grande qu'une surface obtenue par une projection parallèle de l'électrode de mesure (2, 18) et recouvre celle-ci.

15. Unité de détection selon une des revendications 1 à 14, **caractérisée en ce que** l'électrode écran (4, 19) possède une surface active au moins exactement aussi grande que l'électrode de mesure (2, 18).

16. Machine pour la fabrication d'articles en maille comprenant au moins un poste de formation des mailles (29), des moyens (33, 34) pour amener un matériau fibreux (1) étiré au poste de formation des mailles (29) et une unité de détection (26) pour surveiller la qualité du matériau fibreux (1), **caractérisée en ce que** l'unité de détection (26) est réalisée selon au moins une des revendications 1 à 15.
